# EUROPEAN PATENT APPLICATION

(11) **EP 1 325 753 A2**
(43) Date of publication of application: **09.07.2003**
(21) Application number: 02258832.1
(22) Date of filing: 20.12.2002
(51) Int. Cl.: A61K 47/38, A61K 47/34, A61K 47/32, A61K 31/4402

(54) **Bio-compatible means for controlled drug delivery to tissue and method of use**

(30) Priority: 21.12.2001 US 29506
(71) Applicant: ETHICON, INC., Somerville, NJ 08876 (US)
(72) Inventor: Jampani, Hanuman, Bridgewater, NJ 08807 (US); Pendharkar, Sanyog, Old Bridge, New Jersey 08857 (US)
(74) Representative: Mercer, Christopher Paul

(57) **Abstract**

There are provided bio-compatible means for delivery of at least one pharmaceutically active agent to a patient in need of same, comprising a bio-compatible, bio-degradable anionic or cationic carrier and at least one pharmaceutically active agent wherein the agent is cationic when the carrier is anionic and is anionic when the carrier is cationic. The delivery means further comprises at least one bio-compatible enclosing means for the carrier. This enclosing means may be bio-degradable or non bio-degradable and have a predetermined permeation gradient for the passage therethrough of the at least one pharmaceutically active agent. In an alternate embodiment the enclosing means may be biodegradable without a predetermined permeation gradient for the passage therethrough of the at least one pharmaceutically active agent. In all embodiments the active agent is ionically linked to the carrier and the carrier/active agent combination is enclosed in the enclosing means. There is also provided a method of administering at least one pharmaceutically active agent to the tissue surface of a subject in need of same at a rate dependent on the permeability and/or biodegradability of the enclosing means of the delivery means which comprises contacting the tissue surface with a bio-compatible delivery means as described above.

## Description

### Field of the Invention

Bio-compatible means for controlled drug delivery to tissue and method of use of same.

### Discussion of the Prior art

Problems associated with the healing of wounds that are often associated with surgery, including, but not limited, to post-surgical adhesion, infection, and localized pain, are well-known. Many solutions to such problems have been advanced, many of which are discussed hereinbelow. While certain components utilized in the present invention have been employed in suggested solutions to these problems, none of them take the form of nor suggest the present invention.

Rogers et al. U.S. 5,891,460 relates to a method of reducing or preventing post-surgical adhesion formation. The active agent in this disclosure is the anti-asthmatic ketotifen. Rogers et al. teaches that the active agent is administered to the wound site in a number of ways, including administration in conjunction with anionic carbohydrate polymers which form absorbable mechanical barriers to which the ketotifen may be covalently or non-covalently bonded. Rogers et al. also teaches that a particularly suitable mechanical barrier, in conjunction with which the ketotifen is administered, is oxidized regenerated cellulose (ORC). The active agent may also be administered in the form of a film comprising the ketotifen and a bio-polymer. Rogers et al. does not disclose the combination of a bio-active agent with ORC and a biodegradable polymer.

WO00/33893 discloses the ionic bonding of certain therapeutically active peptides to ORC, but makes no mention of controlling the release of the peptides by covering the ORC with a film of another substance.

WO96/40090 also relates to the problem of post-surgical adhesion formation between tissues. More particularly, WO96/40090 discloses various modes for delivery of a 5-lipoxygenase inhibitor associated with either a biodegradable polymer or an absorbable mechanical barrier comprising ORC.

WO97/38737 discloses the combination of ORC with a vitamin E blend and, under certain circumstances, a bio-active material, such as a pain reliever (e.g., benzocaine) to provide a hemostatic composition which can be molded to conform to the contours of the wound. WO97/38737 does not, however, disclose the sustained or controlled release and/or delivery of a bio-active material.

EP0649662 teaches the utilization of ORC as a support layer for a bio-compatible, biodegradable polymer which may contain a biologically active agent. It should be noted that, in this disclosure, a biologically active agent is incorporated in the polymer implant precursor and is not associated with the ORC.

EP0636378 discloses chemo-therapeutic agents comprising a collagen matrix that is reinforced with a bio-absorbable layer, such as polylactic acid (PLA) or polyglycolic acid (PGA) on the one hand, or ORC on the other hand. There is no disclosure of a combination of ORC and PLA and/or PGA.

EP0562864 discloses a bio-absorbable heteromorphic sponge comprising a first and a second bio-polymer component with a pharmacologically active agent added to either component and the second component added to the first component. EP0562864 does not disclose controlled or sustained release of the pharmacologically active agent. Nor does EP0562864 teach or suggest that the pharmacologically active agent is or might be ionically bonded to either the first or second bio-polymer component.

WO00/04939 relates to a dental pin for insertion into a tooth cavity comprising, inter alia, a combination of anionic and cationic carriers and bio-active materials, for example, ORC-chitosan antibiotic combinations. WO00/04939, however, discloses nothing about controlled release of the bio-active materials, nor about coatings or films to control such release.

### Summary of the invention

There are provided bio-compatible means for delivery of at least one pharmaceutically active agent to a patient in need of same, comprising a bio-compatible, biodegradable anionic or cationic carrier and at least one pharmaceutically active agent wherein the agent is cationic when the carrier is anionic and is anionic when the carrier is cationic. The terms anionic and cationic respectively are also intended to include carriers or agents which assume an acidic or basic character when in contact with water. That is to say, suitable carriers and agents may actually exist in the un-ionized state prior to contact with a potential partner of opposite charge. The bio-compatible delivery means further comprises at least one bio-compatible enclosing means for the carrier. It is further contemplated that the devices of the present invention further include at least one carrier layer located on an outer surface of the enclosing means, and suitably, such carrier layers are located on each surface of the enclosing means.

The enclosing means may be biodegradable or non-biodegradable and have a predetermined permeation gradient for the passage therethrough of at least one pharmaceutically active agent. In an alternate embodiment, the enclosing means may be biodegradable, but without a predetermined permeation gradient. In all embodiments the active agent is ionically linked to the carrier and the resulting carrier/active agent combination is enclosed in the enclosing means.

It is preferable that the carrier is an anionic carrier and the active agent is a cationic agent. Suitable classes of agents include, but are not limited to, analgesics, antibiotics, antimicrobials, antivirals, antiinflamatory agents, anticholinergics, antidepressants, antihistamines, antidiabetics, anticonvulsants, antimigranes, antineoplastics, antimalerials, immunisuppressants, cardiovascular drugs and hemostatic agents.

An oxidized regenerated cellulose carrier (ORC) is suitable as the anionic carrier, in particular, in the form of a fabric. Since this material is known to have hemostatic effects, the presence of one or two layers of ORC to the outer surfaces of the enclosing means will improve the hemostatic properties of the bio-compatible delivery means as a whole, in addition to its pharmacologically active properties.

The enclosing means, when permeable, whether biodegradable or otherwise, is in the form of a polymer, preferably a microporous polymer wherein the pore diameter is between 0.01 and 1000 microns or 0.1 and 500 microns, suitably between 0.1 and 50 microns, more suitably between 0.1 and 5 microns, and most suitably between 0.1 and 1 microns. While the enclosing means may be made of any bio-compatible polymer, it is particularly desirable that the enclosing means be made of a film, suitably formed as an envelope made from a polymer selected from the group consisting of polylactic acid or polyglycolic acid (PLA or PGA), mixtures thereof and copolymers of the constituent monomers thereof.

There is also provided a method of administering at least one pharmaceutically active agent to the tissue surface of a subject in need of same, at a rate dependent on the permeability and/or biodegradability of the enclosing means, which comprises the step of contacting the tissue surface with the bio-compatible delivery means of the present invention.

The administration may be purely topical, in which case the enclosing means may or may not be bio-degradable, or within a surgical incision which is then closed, in which case it is preferable, but not essential, that the enclosing means is biodegradable. If a surgical insert is not biodegradable, the incision will have to be opened to remove it, which some procedures require in any case.

While the invention is not limited thereto it is contemplated that the invention will find principal use in the administration of antibiotics or analgesics at the site of surgical procedures or wounds. In the case of analgesics, in particular, use of microporous enclosing means is particularly suitable, since it provides immediate and steady reduction of pain at the wound site, which is always desirable in surgical procedures.

### Brief Description of the drawings

The present invention is described in detail hereinafter and is illustrated with reference to the Figures, in which:
Figure 1 is an elevational, cross-sectional, schematic side view of a prior art device,
Figure 2 is an elevational, cross-sectional, schematic side view of a first embodiment of the present invention,
Figure 3 is an elevational, cross-sectional, schematic side view of a second embodiment of the present invention,
Figure 4 is an elevational cross-sectional, schematic side view of a third embodiment of the present invention,
Figure 5 is a schematic flow diagram of a sample preparation procedure for applying bio-active material to a bio-compatible biodegradable carrier,
Figure 6 is a plot of residual bupivacaine, expressed as percentage by weight of the carrier fabric, against incubation time in hours, comparing uncoated (but bupivacaine carrying) fabric with this fabric coated (but not enveloped) with PLA/PGA,
Figure 7 is a plot of residual bupivacaine, expressed as percentage by weight of the carrier fabric, against incubation time, in hours for bupivacaine-carrying fabric enveloped with perforated polypropylene film, and

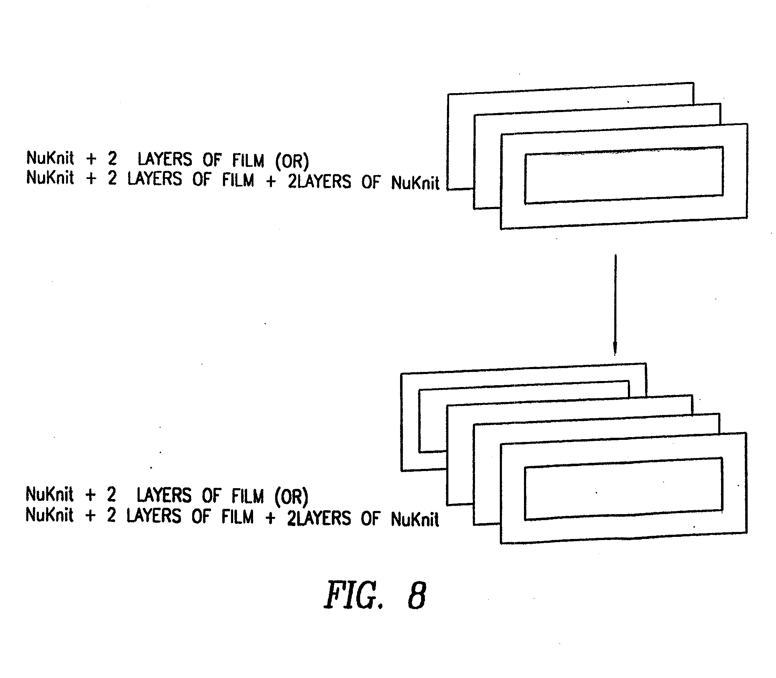

Figure 8 is a schematic flow diagram of the sample preparation procedure for enclosing the bio-active material on a bio-compatible biodegradable carrier with a polymeric envelope.

### Description of the Preferred Embodiments

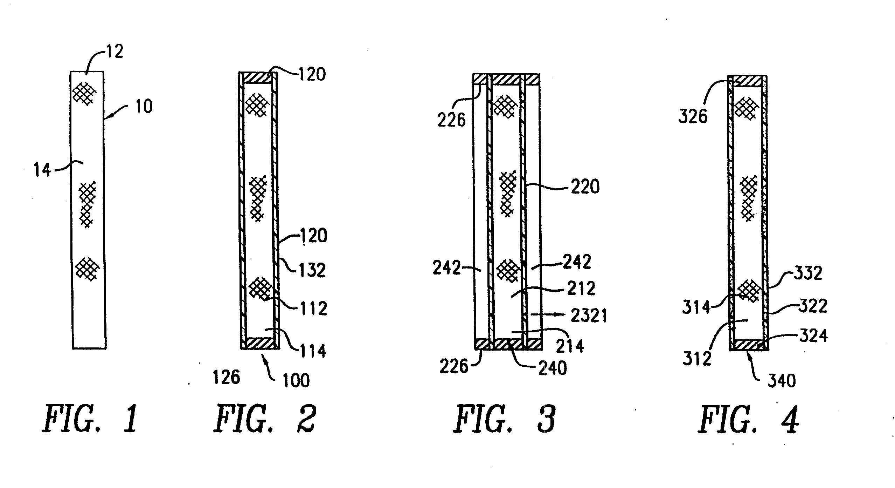

As shown in Figure 1, prior art delivery means 10 comprising a bio-compatible biodegradable carrier 12 having a bio-active agent 14 attached thereto is known in the art. This prior art delivery means 10 is designated item NKD1.

At first embodiment of the bio-compatible delivery means 110 of the present invention is shown in Figure 2 and similarly comprises a bio-compatible biodegradable carrier 112 having a bio-active agent 114 14 attached thereto The carrier 112 having the agent 114 is further enclosed within a bio-compatible biodegradable enclosing means, such as an envelope 120, which is peripherally sealed at edge 126. This first embodiment of the delivery means 110 releases the bio-active agent 114 when the patient's body fluids degrade the envelope 120. The envelope 120 may further have micropores 132, such as perforations, of predetermined size.

A second embodiment of the bio-compatible delivery means 210 of the present invention, shown in Figure 3, similarly comprises a bio-compatible biodegradable carrier 212 having a bio-active agent 214 14 attached thereto and both being enclosed within a bio-compatible biodegradable enveloping means, such as an envelope 220, which includes micropores 232, such as perforations, of predetermined size. The envelope 220 has located on at least one outer surface thereof, preferably on both outer surfaces, a further layer of bio-compatible biodegradable carrier 242, which is similar to the inner layer 212, but is not bonded to any bio-active agent 214. All layers are preferably sealed together peripherally, for example, by heat sealing at edge 226. This embodiment suitably utilizes an envelope made of PLA, PGA, or copolymers thereof.

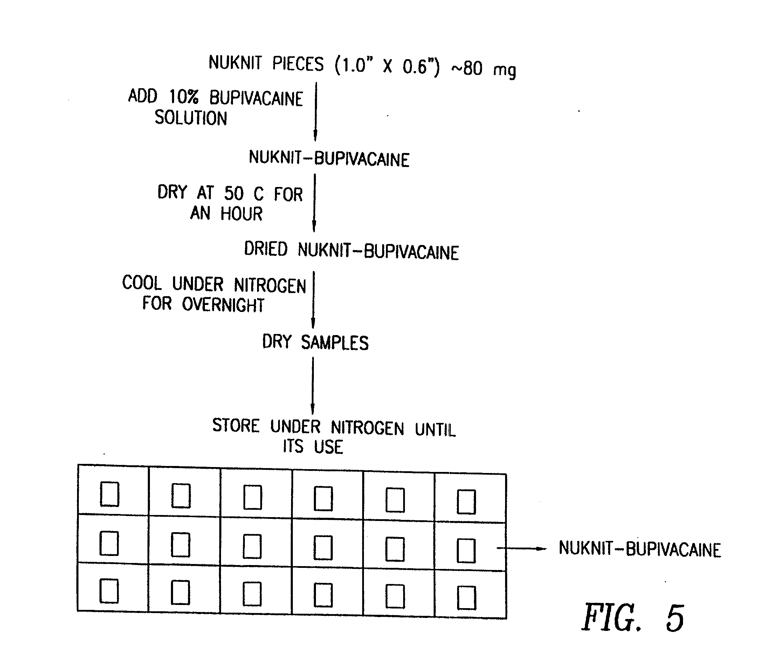

Figure 4 shows a third embodiment of the bio-compatible delivery means, 310 of the present invention, which similarly comprises a bio-compatible biodegradable carrier 312 having a bio-active agent material 314 absorbed thereon. The carrier 312 and the agent 314 are enclosed within a bio-compatible, but not biodegradable, enclosing means, such as an envelope 320, which includes micropores, such as perforations 332, of predetermined size. All layers are preferably sealed together peripherally, suitably by heat sealing at edge 326. This embodiment is especially useful when used purely topically, that is to say, when it is not placed in a location from which it cannot be removed without surgical incision. This third embodiment preferably utilizes an envelope made of polyethylene or polypropylene.

### Example 1

### Preparation 'Five -Layered Device' for Pain Management.

A stock solution is prepared by dissolving Bupivacaine (Marcaine®) free base in a 60/40 aq. iso-propyl alcohol (IPA) solvent, until a clear solution is obtained, such that the final concentration is about 5%. A 1g piece of prewashed Nu-Knit fabric (which, as know to those having skill in the art, is bio-compatible and bio-degradable) is soaked with 2.5 ml of this stock solution. Nu-Knit is commercially available from Ethicon, of Somerville, New Jersey. The wet piece of Nu-Knit fabric is then placed in a pre-heated oven at 50°C for 1 hour. It is then stored under a continuous flow of dry nitrogen gas. This drug-loaded fabric is designated NKD1

Prior to use, the drug-loaded fabric ('NKD1': - Nu-Knit Marcaine-individually loaded) is equilibrated to ambient conditions for an hour. PLA, PGA-based film (which, as known to those having skill in the art, is bio-compatible and bio-degradable), approximately 50 microns thick is cut into pieces appropriately, such that it completely encases the NKD1 sample, and is then peripherally heat sealed. This drug-loaded fabric composite is designated NKD2

In a modification of the above step, a plurality of small holes are created for example, by perforating with a needle, in the PLA/PGA film. These holes are approximately 400-500 microns in diameter. The film is then covered by two additional pieces of Nu-Knit (which are not drug-coated) on both sides, resulting in a 5-layed delivery means.

The entire 5-layered delivery means is then sealed at the edges ensuring that the innermost NKD1 layer remains untouched. The sealing is performed using a pre-heated sealer, at or near the softening temperature of the polymer (e.g., approximately 100°C for PLGA-65/35), for 4-6 seconds at each edge. This drug-loaded fabric composite is designated NKD3.

Utilizing microporous PLA/PGA film with 10 micron pores, a fabric composite similar to NKD3, designated NKD5 is obtained

In another embodiment, in place of the PLA/PGA envelope of NKD2, there is utilized an envelope made of microporous polypropylene film (which, as known to those having skill in the art, is bio-compatible. This drug-loaded fabric composite is designated NKD4.

In vitro release testing of the above-identified drug loaded composite fabrics was carried out and is summarized in the table below.

| Time / % Drug Release | | | | | |
|---|---|---|---|---|---|
| | NKD1 | NKD2 | NKD3 | NKD4 | NKD5 |
| 6 hours | 104 | 1.31 | N/A | 2.17 | |
| 24 hours | 99.8 | 0.6 | 27.1 | 25.4 | 4.7 |
| 48 hours | | | | | 14.6 |
| 72 hours | 93.8 | 2.2 | 70.2 | 78.04 | 28.4 |

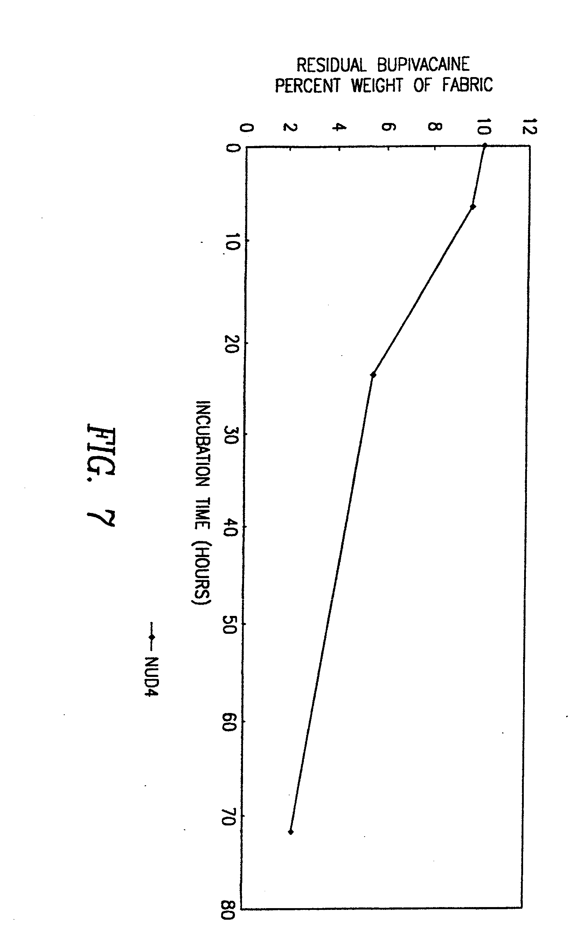

The data for NKD4 set forth in the table above, is expressed differently in Figure 7, wherein the residual bupivacaine residue is expressed as percentage, by weight, of the carrier fabric (NKD1) against incubation time, in hours.

### Example 2

In accordance with the procedures of Example 1, there may be prepared composites of the structure of NKD2 and/or NKD3, wherein the perforations are microporous perforations in the range of 0.01-100 microns in diameter. In additional, in place of bupivacaine, there may be utilized any tissue compatible bio-active agent, including, but not limited to, analgesics, antibiotics, antimicrobials, antivirals, antiinflamatory agents, anticholinergics, antidepressants, antihistamines, antidiabetics, anticonvulsants, antimigranes, antineoplastics, antimalerials, immunisuppressants, cardiovascular drugs, anti-adhesive agents, vasoconstrictors, growth factors (PDGF), and hemostatic agents, suitably, gentamicin, ofloxacin, silver, verapamil miconazole, ketoconazole, taxol, vincristine and vinblastine.

In addition, a sample listing of suitable pharmaceutically active agents is set forth in U.S. Patent No. 6,255,502, which is hereby incorporated herein by reference.

In place of PLA/PGA, there may be utilized any bio-compatible and biodegradable, formable, heat sealable polymer film such as. polylactides, polyglycolides, polycapralactones, polyanhydrides, polyamides, polyurethanes, polyesteramides, polyorthoesters, polydioxanones, polyacetals, polyketals,polycarbonates, polyorthocarbonates, polyphosphazenes, polyhydroxybutyrates, polyhydroxyvalerates, polyalkyleneoxalates, polyalkylenesuccinates, poly (maleic acid) polymers, polymaleicanhydrides, poly (methylvinyl) ethers, poly (amino acids), chitin, chitosan, gelatin and copolymers, terpolymers, or combinations or mixtures of the above materials.

In place of polypropylene, there may be utilized polyethylene or any bio-compatible formable, heat sealable polymer film, but which is not biodegradable.

### Example 3

### Dip coating of ORC/Bupivacaine Matrix

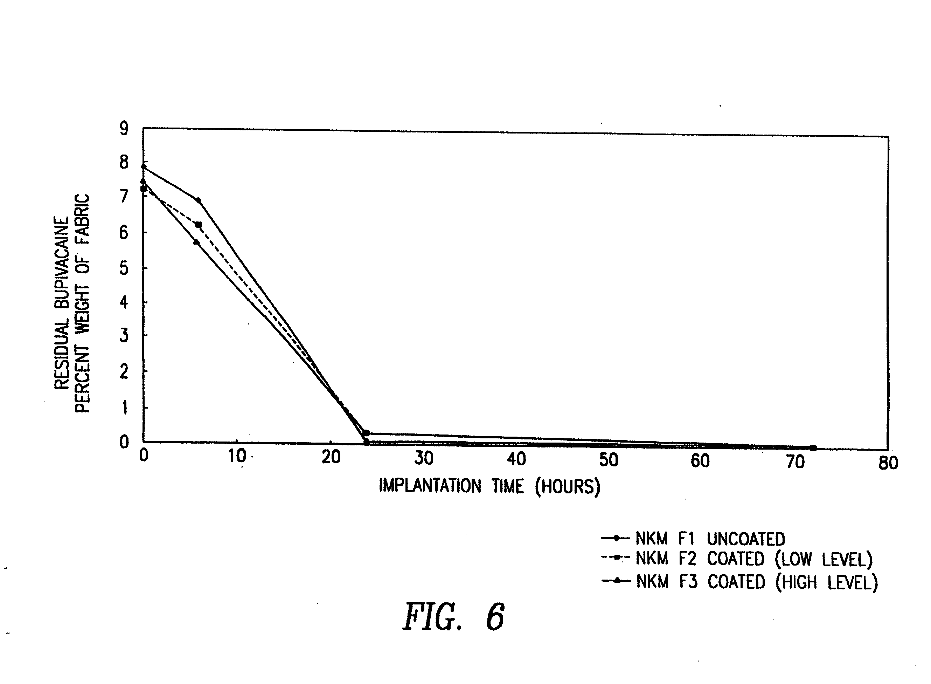

A matrix of NKD1 was coated with a mixture of PLA and PLG by dipping the matrix into a solution containing 5 and 50 wt % of the polymers dissolved inethyl acetate or methylene chloride. The product was then air dried and tested *in vitro* in accordance with the procedures of Example 4 below. The *in vivo* test results are shown in Figure 6. It is noted that there was no substantial difference in the release time and amount, between the uncoated NKD1 and the corresponding dip coated samples.

### Example 4

### Determination of Bupivacaine (Marcaine) Release from ORC-Matrix

The ORC-active agent combinations of example 2 and 3 can be suspended in neutral buffers at pH 7.4 at 37°C to simulate body conditions for the purpose of studying the release of the drug in the buffer (in vitro). The test samples released Marcaine in the buffer while degrading. The ORC matrix is expected to degrade completely in 3-5 days if needed, degradation can be accelerated (terminal samples) by adding sodium bicarbonate base to the buffer solution.

More particularly, for example, water bath is pre-heated to 37°C. Test samples of the ORC-Marcaine are cut into small pieces and the samples (in the range of 0.05g - 01g) are weighed and placed in labeled vials. The desired amount of buffer (e.g., 100, 50, 25 or 10 times, by weight, of the test sample) is added. For terminal samples, sufficient sodium bicarbonate is added to make a 0.15 M solution. The vials are placed in the pre-heated water bath for the desired period of time (approximately 10 min-120 hours).

The vials are removed at the specified time intervals and the solution is quickly drained in a disposable syringe fitted with a 0.45-um filter. The solution is filtered through and collected in appropriately labeled vials and frozen just prior to high pressure liquid chromatography (HPLC) analysis

Based on the HPLC results that are obtained, in units of (µg/g) of Marcaine in the terminal solution, the total amount of Marcaine deposited and released after total degradation of the test sample is calculated. Using the data obtained for the concentration of Marcaine in the solution at any specific time interval, and from the total amount, the percentage release is assessed, and the percentage release, as a function of the time intervals, is plotted.

## Claims

1. Bio-compatible means for delivery of at least one pharmaceutically active agent to a patient in need of same comprising:
a) a bio-compatible, biodegradable anionic or cationic carrier,
b) at least one pharmaceutically active agent wherein said agent is cationic when the carrier is anionic and is anionic when the carrier is cationic,
c) at least one bio-compatible enclosing means having at least one outwardly directed surface, said active agent being ionically linked to said carrier, thereby forming a carrier/active agent combination, said carrier/active agent combination being enclosed in said enclosing means.

2. The bio-compatible means of claim 1, wherein said at least one outwardly directed surface of said enclosing means has a predetermined permeation gradient for the passage therethrough of said at least one pharmaceutically active agent.

3. The bio-compatible means of claim 1 or claim 2, wherein said enclosing means is biodegradable.

4. The bio-compatible means of any one of claims 1 to 3, wherein the carrier is an anionic carrier.

5. The bio-compatible means of claim 4, wherein the anionic carrier is an oxidized regenerated cellulose carrier.

6. The bio-compatible means of claim 5, wherein the anionic carrier is an oxidized regenerated cellulose fabric.

7. The bio-compatible means of any one of claims 4 to 6, wherein the active agent is a cationic agent.

8. The bio-compatible means of claim 7, wherein the active agent is a cationic analgesic, antibiotic, antimicrobial, antiviral, anti-inflammatory agent, anticholinergic, antidepressant, antihistamine, antidiabetic, anticonvulsant, antimigraine, antineoplastic, antimalarial, immunosuppressant, cardiovascular drug, growth factor or hemostatic agent.

9. The bio-compatible means of any one of claims 1 to 8, wherein the enclosing means is a polymer film.

10. The bio-compatible means of claim 9, wherein the polymer film is microporous.

11. The bio-compatible means of claim 10, wherein said polymer is a microporous polymer having a pore size of between 0.01 and 1000 microns, preferably between 0.1 and 500 microns, more preferably between 0.1 and 50 microns, even more preferably between 0.1 and 5 microns, and most preferably between 0.1 and 1 microns.

12. The bio-compatible means of any one of claims 1 to 11, wherein the enclosing means is a polymer film comprising PLA, PLG, a mixture thereof or a copolymer of the constituent monomers thereof.

13. The bio-compatible means of any one of claims 1 to 11, wherein the enclosing means comprises polyethylene, polypropylene, a mixture thereof or a copolymer of the constituent monomers thereof.

14. The bio-compatible means of any one of claims 1 to 13, additionally comprising at least one further carrier layer located on at least one outwardly facing surface of said enclosing means.

15. The bio-compatible means of any one of claims 1 to 14 for use in therapy.
